# EUROPEAN PATENT SPECIFICATION

(11) **EP 2 672 936 B1**
(45) Date of publication and mention of the grant of the patent: **13.12.2017**
(21) Application number: 12745296.9
(22) Date of filing: 08.02.2012
(51) Int. Cl.: A61F 5/01, A61H 1/02

(54) **A DEVICE AND METHOD FOR THE TREATMENT OF ADHESIVE CAPSULITIS**
VORRICHTUNG UND VERFAHREN ZUR BEHANDLUNG VON ADHÄSIVER CAPSULITIS
DISPOSITIF ET PROCÉDÉ DE TRAITEMENT D'UNE CAPSULITE RÉTRACTILE

(30) Priority: 08.02.2011 AU 2011900394
(43) Date of publication of application: 18.12.2013
(73) Proprietor: Alasca Pty Ltd ATF The John Cully Family Trust, Blacktown NSW 2148 (AU)
(72) Inventor: DOYLE, Max, Coogee, NSW 2034 (AU)
(74) Representative: Potter Clarkson LLP
(86) International application number: PCT/AU2012/000114
(87) International publication number: WO 2012/106760

(56) References cited:
- TW-B- 455 488
- US-A- 5 067 479
- US-A- 5 179 939
- US-A- 5 179 939
- US-A- 5 645 521
- US-A1- 2004 087 880
- US-A1- 2010 076 354

## Description

### Field of the Invention

The present invention relates to a device and method for the treatment of adhesive capsulitis. The invention has been developed primarily for use in respect of the treatment of adhesive capsulitis and will be described hereinafter with reference to this application. However, it will be appreciated that the invention is not limited to this application or particular field of use.

### Background

Adhesive capsulitis, or frozen shoulder as it is commonly known, is a disorder in which the shoulder capsule and the connective tissue becomes inflamed and stiff causing chronic pain.

Existing means of treatment may employ anti-inflammatory drugs or the application of heat. In some cases electrical nerve stimulation may be used to reduce pain by blocking nerve impulses. In extreme cases, manipulation of the shoulder under general anesthesia is required to break up the adhesions.

An example of an existing means of treatment is shown in US 2010/0076354 A1 which discloses a shoulder continuous passive motion (CPM) device. The CPM device has a motor and a drive mechanism that is configured to move a slidable arm holder linearly back and forth. A user can insert at least a portion of his arm into the arm holder such that the CPM device moves his arm linearly back and forth.

However, existing arrangements suffer from numerous disadvantages, including being ineffective, painful and requiring the presence of medical staff.

As such, a need therefore exists for a device and method for the treatment of adhesive capsulitis that is effective, less painful and may be performed without the supervision of medical staff. It is to be understood that, if any prior art information is referred to herein, such reference does not constitute an admission that the information forms part of the common general knowledge in the art, in Australia or any other country.

### Summary

An object of the claimed invention is to provide a device for the treatment of adhesive capsulitis which will overcome or substantially ameliorate at least some of the deficiencies of the prior art, or to at least provide an alternative.

According to one aspect, there is provided a device for the treatment of adhesive capsulitis in a joint of a patient, the device comprising the features recited in Claim 1.

Advantageously, the device may be provided to patients suffering from adhesive capsulitis. The manipulation effector manipulates the joint so as to break up the adhesions surrounding the joint. The device is a portable device allowing for home treatment, advantageously allowing the patient to maintain certain treatment regimens, such as upon awakening and before going to bed.

Preferably, the manipulation effector is mechanically coupled to the driven oscillator.

Advantageously, the driven oscillator may be configured such that the characteristics of the point-to-point motion profile provide the most effective treatment. In some embodiments, the manipulation effector is adapted to move in a point-to-point motion profile that may be varied according to certain parameters, such as the physical characteristics of the patient, progress of treatment and the like. Further advantageously, the mechanical coupling between the manipulation effector and driven oscillator can be chosen according to the operational requirements of the device, such as the weight, power consumption and power output of the device.

Preferably, the manipulation effector is adapted to move in a point-to-point motion profile comprising a displacement of between 3mm and 12mm. Preferably, the manipulation effector is adapted to move in a point-to-point motion profile comprising a displacement of between 5mm and 10mm. Preferably, the manipulation effector is adapted to move in a point-to-point motion profile comprising a displacement of about 7.5mm. Preferably, the manipulation effector is adapted to move in a point-to-point motion profile comprising a period of between 0.015s and 0.3s. Preferably, the manipulation effector is adapted to move in a point-to-point motion profile comprising a period of between 0.016s and 0.9s. Preferably, the manipulation effector is adapted to move in a point-to-point motion profile comprising a period of between 0.017s and 0.15s.

Advantageously, the amplitude and frequency of the point-to-point motion profile are chosen so as to most effectively treat the joint.

Advantageously, a relatively simple mechanical arrangement may be employed to displace the manipulation effector in accordance with the point-to-point profile.

Preferably, the manipulation effector is adapted to move in a point-to-point motion profile comprising at least one phase of motion comprising a jerk motion.

Advantageously, the jerk motion further enhances the effectiveness of the treatment.

Preferably, the manipulation effector is adapted to move in a point-to-point motion profile comprising:
a first phase of motion under increasing acceleration;
a second phase of motion under constant acceleration;
a third phase of motion under decreasing acceleration; and
a fourth phase of motion under zero acceleration.

Preferably, the manipulation effector is adapted to move in a point-to-point motion profile comprising:
a fifth phase of motion under increasing deceleration;
a sixth phase of motion under constant deceleration; and
a seventh phase of motion under decreasing deceleration.

Advantageously, the seven phases of motion impart a jerk motion upon the manipulation effector.

The device comprises a body and an anchor connected to the body and adapted for securement to a secured object.

Advantageously, in use the device may be attached to an secured object using the anchor. For example, in one embodiment, the device may be attached to a doorknob by a cord fastened to the anchor. The manipulation effector, may be coupled to the driven oscillator at a distal end of the anchor so as to manipulate the joint. In this manner, the limb of the patient may be placed in tension during treatment. Further advantageously, the

Preferably, the manipulation effector is adapted for grasping by a hand of the patient.

Advantageously, no attachment means are required to manipulate the limb. The patient may grasp the manipulation effector during treatment and may release the manipulation effector should any sever pain be experienced.

Preferably, the manipulation effector comprises releasable attachment means adapted for attachment to a limb of the patient.

Advantageously, the manipulation effector may be attached to the patient, perhaps by a releasable cuff adapted to engage the wrist of the patient.

Preferably, the drive shaft comprises a bearing surface having an axis offset from the axis of the drive shaft and wherein the crank bears against the bearing surface. Advantageously, a powered motor such an electric motor may be used to drive the driven oscillator. Such a motor may provide the necessary power while not adversely affecting the portability of the device. Furthermore, the power to the motor or the configuration of the crank may be varied to characterise the point-to-point motion profile. The manipulation effector is adapted to move in a point-to-point motion profile comprising:
a first phase of motion under constant acceleration;
a second phase of motion under zero acceleration; and
a third phase of motion under constant deceleration.

### Brief Description of the Drawings

Notwithstanding any other forms which may fall within the scope of the present invention, preferred embodiments of the invention will now be described, by way of example only, with reference to the accompanying drawings in which:
Fig. 1a shows a block diagram of the functional components of a device for the treatment of adhesive capsulitis in a joint of a patient in accordance with a first preferred embodiment of the present invention;
Fig. 1b shows a block diagram of the functional components of a device for the treatment of adhesive capsulitis in a joint of a patient in accordance with second preferred embodiment of the present invention;
Fig. 2 shows exemplary point-to-point motion profiles in accordance with preferred embodiments of the present invention;
Fig. 3 shows the mechanical arrangement of third embodiment of the device as shown in Fig. 1a, not covered by the invention;
Fig. 4 shows a method of treatment for adhesive capsulitis in a joint of a limb of a patient;
Fig. 5 shows a top view of the device in accordance with a fourth preferred embodiment of the present invention;
Fig. 6 shows a side view of the device of Fig. 5 in accordance with the fourth preferred embodiment of the present invention;
Fig. 7 shows a side view of the device of Fig. 5 in use in accordance with the fourth preferred embodiment of the present invention;
Fig. 8 shows a bottom view of the device of Fig. 5 in accordance with the fourth preferred embodiment of the present invention;
Fig. 10, there is shown an internal view of the device of Fig. 5 in accordance with the fourth preferred embodiment of the present invention;
Fig. 11, there is shown a controller for remote control of the device of Fig. 5 in accordance with the fourth preferred embodiment of the present invention;
Fig. 12 shows a perspective view of the controller of Fig. 11 in accordance with the fourth preferred embodiment of the present invention;
Fig. 13 shows a top view of the controller of Fig. 11 in accordance with the fourth preferred embodiment of the present invention; and
Fig. 14 shows a bottom view of the controller of Fig. 11 in accordance with the fourth preferred embodiment of the present invention.

### Detailed Description of Specific Embodiments

It should be noted in the following description that like or the same reference numerals in different embodiments denote the same or similar features.

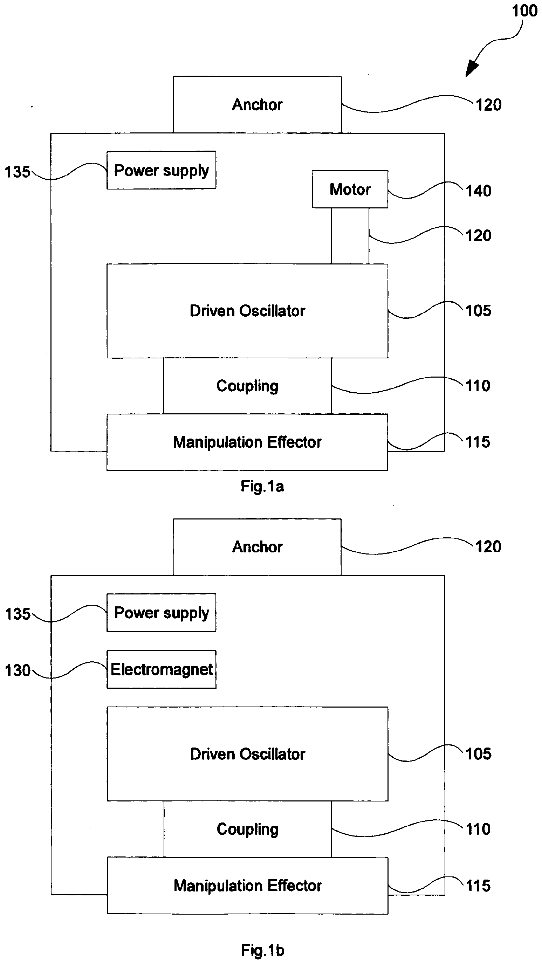

Figs. 1a and 1b shows a block diagram of the functional components of a device 100 for the treatment of adhesive capsulitis in a joint of a patient. In other embodiments, the device 100 may be suited to the treatment of other disorders, such as disorders similar to adhesive capsulitis, including calcific tendinitis and the like. The device 100 comprises a driven oscillator 105 coupled to a manipulation effector 115 by a coupling 110.

The manipulation effector 115 is adapted to manipulate the joint so as to treat the adhesive capsulitis of the joint. The manipulation effector 115 typically manipulates the limb of the patient comprising the joint, but may also, in other embodiments, manipulate the joint directly, by applying force directly to the joint. As such, the manipulation effector 115 may be adapted to attach to the limb of the patient, for example by way of a releasable cuff secured in place by hook and loop fasteners. In other embodiments, the manipulation effector 115 is adapted to be grasped by a hand of the patient. In further embodiments the manipulation effector 115 does not attach to the limb of the patient, but merely bears upon the limb so as to apply force to the limb.

The driven oscillator 105 is adapted to displace the manipulation effector 115 in accordance with a point-to-point motion profile. The point-to-point motion profile may be chosen to maximise the treatment of the adhesive capsulitis. Various mechanical arrangements, some of which are described herein, may be employed to couple the driven oscillator 105 to the manipulation effector 115 to displace the manipulation effector 115 in accordance with a particular point-to-point motion profile.

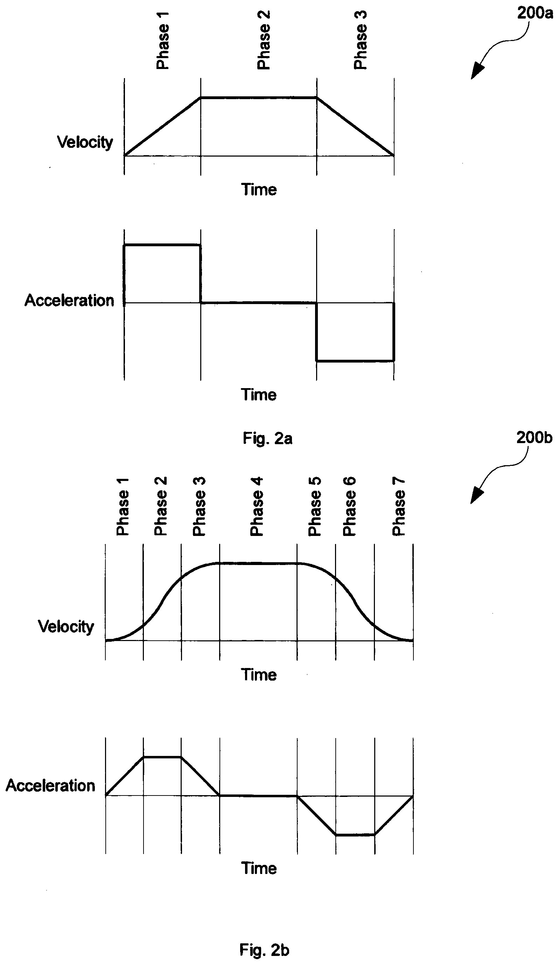

Fig. 2a shows a first exemplary point-to-point motion profile 200a. The point-to-point motion profile 200a may be realized using a relatively simple mechanical arrangement. Specifically, as shown in the acceleration plot, the point-to-point motion profile 200a comprises a first phase of motion under constant acceleration, a second phase of motion under zero acceleration and a third phase of motion under constant deceleration. As is shown in the velocity plot of the point-to-point motion profile 200a, the manipulation effector 115, starting from rest, linearly increases in velocity during the first phase. During the second phase, the manipulation effector 115 experiences zero acceleration and travels at constant velocity. During the third and final phase, the manipulation effector 115 experiences deceleration and the velocity of the manipulation effector 115 decreases in a linear fashion until the manipulation effector 115 comes to rest.

Fig. 2b shows a second exemplary point-to-point motion profile 200b. The point-to-point motion profile 200b comprises at least one phase of motion comprising a jerk motion. The jerk motion further increases the effectiveness of the treatment of adhesive capsulitis. In the embodiment shown in Fig. 2b, the point-to-point motion profile 200b comprises one or more phases of motion, comprising a first phase of motion under increasing acceleration, a second phase of motion under constant acceleration, a third phase of motion under decreasing acceleration, a fourth phase of motion under zero acceleration, a fifth phase of motion under increasing deceleration, a sixth phase of motion under constant deceleration and a seventh phase of motion under decreasing deceleration.

In one embodiment, the point-to-point motion profile comprises between 200-4,000 strokes per minute and preferably, between 400-3,500 strokes per minute

In one embodiment, the point-to-point motion profile has a displacement of between 3mm and 12mm. In a more specific embodiment, the point-to-point motion profile has a displacement of between 5mm and 10mm. In an even further specific embodiment, the point-to-point motion profile has a displacement of about 7.5mm. Furthermore, in one embodiment, the point-to-point motion profile has a period of between 0.015s and 0.3s. In a more specific embodiment, the point-to-point motion profile has a period of between 0.016s and 0.9s. In an even further specific embodiment, the point-to-point motion profile has a period of between 0.017s and 0.15s.

In use, in one embodiment, the patient may be positioned in a treatment position, such as a sitting position or a prone position. The limb of the patient comprising the affected joint may be allowed to hang vertically downwards. The device 100 is then attached to the limb and set in an operational mode. In this manner, the inertia generated by the driven oscillator 105 displace the manipulation effector 115 so as to manipulate the limb.

In an alternative embodiment, the device further comprises an anchor 120 distal to the manipulation effector and adapted for securement to an immovable object, such as a door knob or bolt secured within a wall. In this manner, the device 100, secured by the anchor 120, exerts a resultant force upon the manipulation effector 115.

While the embodiment shown in Figs. 1a and 1b shows the anchor 120, driven oscillator 105 and manipulation effector 115 as being integral, it should be noted that, in various other embodiments, the anchor 120, driven oscillator 105 and manipulation effector 115 may be coupled in various configurations, or located in various places. It should be noted also that the driven oscillator 105 may exert direct force on either the anchor 120 or the manipulation effector 115, such that a resultant force is generated to displace either the manipulation effector 115 or the body of the device 100.

For example, the driven oscillator 105 and anchor 120 may be located away from the manipulation effector 115, perhaps by the driven oscillator 105 being located at and attached to an immovable object by the anchor 120, and connected to the manipulation effector 115, such as a wrist cuff, by a coupling 110, such as a cord. In this manner the driven oscillator 105, located at the immovable object, exerts force on the cord, causing the manipulation effector 115 to maniplulate the joint.

In another example, the driven oscillator 105 and manipulation effector 115 may be integral, with the anchor 120 being attached to an immovable object and the driven oscillator 105 being attached to the anchor 120 by a cord so as to impart a resultant force upon the manipulation effector 115.

In certain embodiments, the manipulation effector 115 is adapted for grasping by a hand of the patient. For example, the manipulation effector 115 may be shaped as a handle, allowing the patient to grasp the manipulation effector 115 during treatment. Alternatively, the manipulation effector 115 may comprise releasable attachment means adapted for attachment to a limb of the patient. For example, the manipulation effector 115 may comprise a cuff adapted to be secured about the limb of the patient, perhaps at the wrist. The cuff may be secured by complimentary hook and loop fastening means.

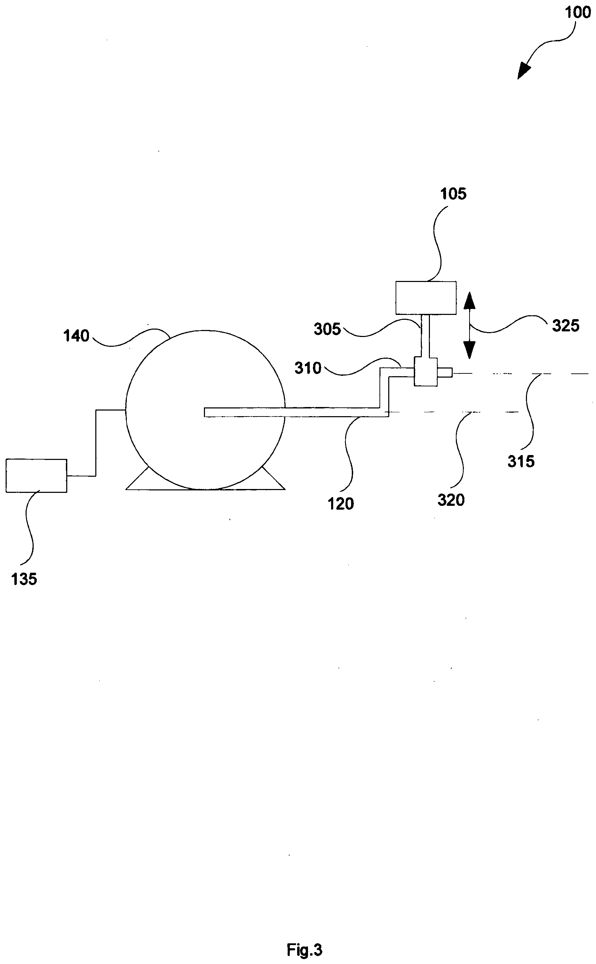

Fig 1a shows a preferred embodiment in which the device 100 comprises a motor 140 coupled to a power supply 135 and adapted to drive the driven oscillator 105. In a further specific embodiment not covered by the invention, Fig. 3 shows the device 100 comprising a drive shaft 120 coupling the motor 145 and the driven oscillator 125. In one particular embodiment, the driven oscillator 105 is mechanically coupled to the drive shaft 120 by a crank 305 adapted to impart a linear reciprocating motion 325 upon the driven oscillator 105 when the motor is in use. Specifically, the drive shaft 140 comprises a bearing surface 310 having an axis 315 offset from the axis 320 of the drive shaft and wherein the crank 305 bears against the bearing surface.

Alternatively, in an embodiment covered by the invention, the driven oscillator 105 is mechanically coupled to the drive shaft 140 at an attachment point offset from the axis of the drive shaft 140 and adapted to impart a rotational motion upon the driven oscillator 105 when the motor is in use.

In one embodiment, the drive shaft 140 may be a lengthy flexible drive shaft 140 encased in a protective sheath. Such an arrangement would allow the motor to be located some distance from the driven oscillator 105, perhaps on the floor of the treatment room.

In this manner, the motor 125 may be an electric motor, perhaps a direct current motor coupled to a direct current power supply, such as a rechargeable battery pack. Alternatively, the motor 125 may be an alternating current motor coupled to an alternating current power supply, such as an electric wall socket.

In an alternative embodiment not covered by the invention, as shown in Fig. 1b, the device 100 further comprises an electromagnet 130 magnetically coupled to the driven oscillator and adapted to impart a linearly reciprocating motion upon the driven oscillator 105 in use. For example, the driven oscillator 105 may be comprised of metal and magnetically coupled to the electromagnet 130. As such, the periodic energisation of the electromagnet 130 may attract or repel the driven oscillator 105, thereby imparting a reciprocating motion upon the driven oscillator 105. In certain embodiments, the device 100 may be provided with both the electromagnet 130 and the motor 140.

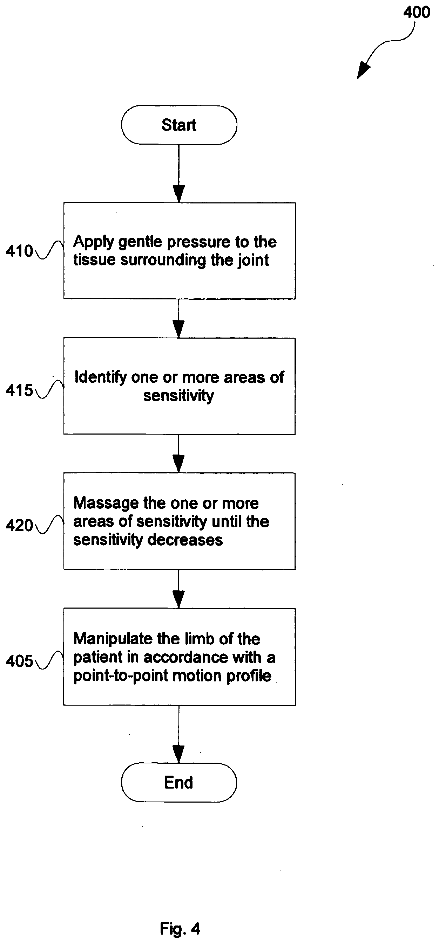

Fig. 4 shows a method 400 of treatment for adhesive capsulitis in a joint of a limb of a patient. The method comprising at least the step 405 of manipulating the limb of the patient in accordance with the point-to-point motion profile 200.

In one embodiment, the step 405 of manipulating the limb of the patient comprises step placing the limb of the patient under longitudinal compression or longitudinal tension to as to further increase the efficiency of the treatment. The patient may be placed in a supine position and the limb manipulated patient in a vertical direction. Alternatively, the patient may be placed in a prone position and the limb manipulated in a vertical direction. Yet further, the patient may be placed in a seated position and the limb manipulated in a horizontal or vertical direction.

Typically, the step of manipulating 405 the limb of the patient in accordance with a point-to-point motion profile is repeated between 1 and 5 times but preferably the step of manipulating the limb of the patient in accordance with a point-to-point motion profile is repeated 3 times. Furthermore, the step 405 of manipulating the limb of the patient comprises the step of manipulating the limb of the patient for a duration of between 0.5 and 10 minutes.

In certain embodiments, the method 400 comprises steps 410 to 420 to treat the adhesion of the muscle tissue surrounding the joint. Specifically, the method 400 comprises step 410 where gentle pressure is applied to the tissue surrounding the joint. At step 415, one or more areas of sensitivity are identified. At step 420 the one or more areas of sensitivity are massaged, typically for between 5 and 40 minutes and preferably for between 15 and 25 minutes, until the sensitivity decreases. Any of steps 410 to 420 may be repeated any number of times.

While step 420 may be performed manually, the device 100 having a driven oscillator 105 and a manipulation effector 115 mechanically coupled to the driven oscillator 105, may be provided to displace the limb in accordance with the point-to-point motion profile. In this manner, the manipulation effector 115 may be attached to the limb of the patient; and the device 100 operated to manipulate the limb.

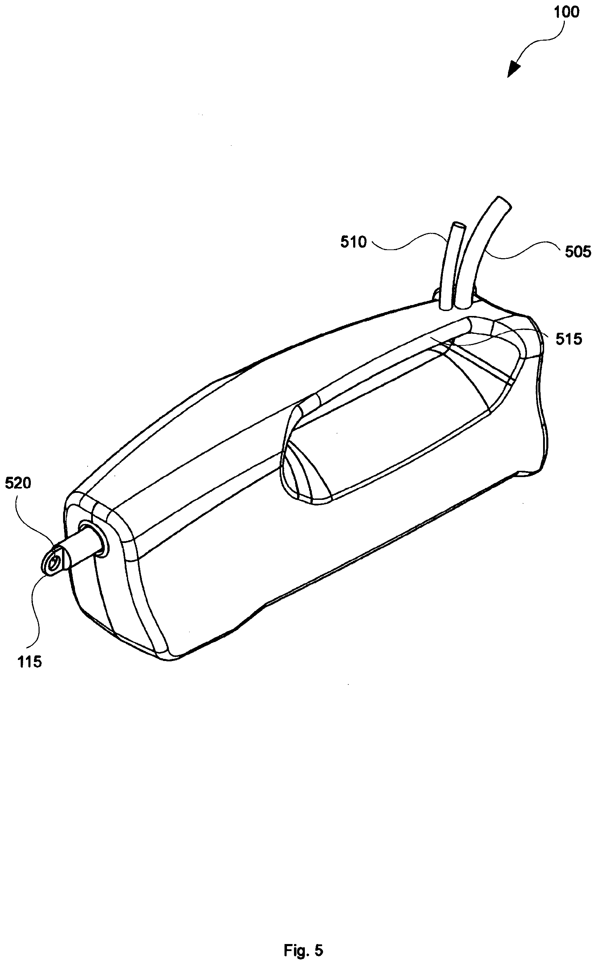

Turning now to Figs. 5 to 14, there is shown an embodiment of the device 100 as an hand-held appliance. In this embodiment, the device 100 is provided with a ruggedized and portable construction so as to be suitable for use in the home, in a physiotherapy practice and the like. Typically, the device 100 will weigh no more than 5kg and comprise a durable plastic outer shell. The device 100 may be battery operated, such as by a lightweight Lithium-ion battery, or powered as is shown in figures, either by AC or DC power.

Fig. 5 shows a top view of the device 100 with the manipulation effector 115 extending therefrom. Herein, the manipulation effector 115 comprises an attachment point, such as a hole 520 in the embodiment shown, for the purposes of attachment of a strap, handle or the like for attachment to a limb of a patient. In this manner, the device 100 may be held by a treatment provider in manipulating the limb of the patient. Also, as will be described in further detail below, the device 100 may further comprise an anchor 120 located distally from the manipulation effector 115 for the purposes of anchoring the device 100 such that no manual handing of the device is required by the treatment provider in use. The anchor 120 further allows the device 100 to be attached to various convenient attachments points, such as a door knob in a house.

The device 100 comprises a flexible power cord 505 and a control cord 510 for a controller as described in further detail below. The device 100 further comprises a handle 515, for carrying the device.

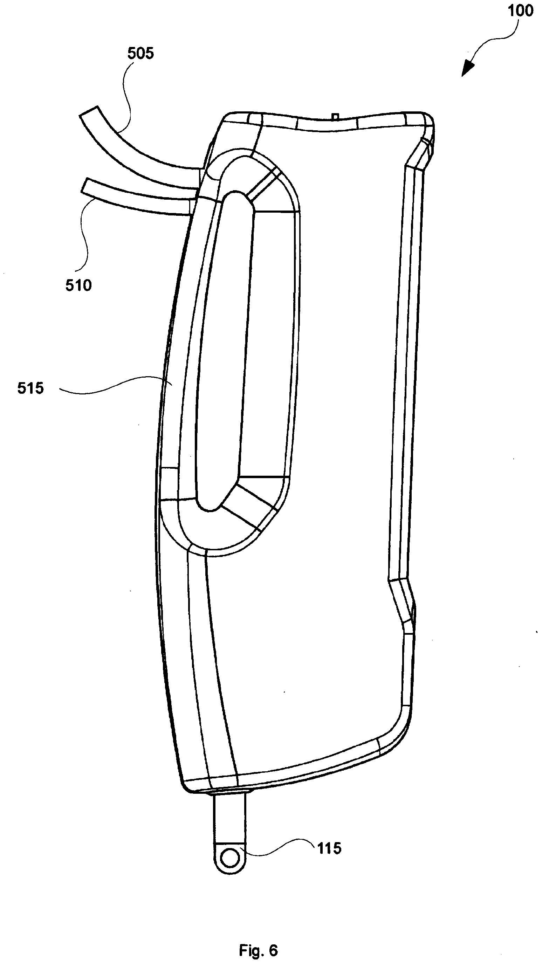

Fig. 6 shows a side view of the device 100 in this embodiment.

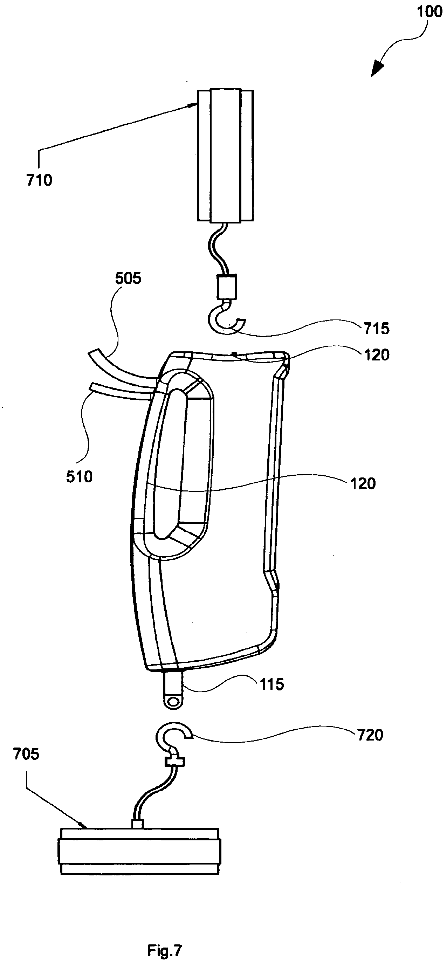

Fig. 7 shows a side view of the device 100 in use, wherein the device 100 is coupled to a patient engagement 705 and an anchor point 710. As alluded to above, the patient engagement 705 may be any suitable means for attachment to the patient but typically attaches to the wrist of the patient (therefore taking on the form of a releasable strap) or is held by the hand of the patient (therefore taking on the form of a handle). Also, the anchor point 710 may be any suitable anchor point, typically located in various orientations, such as at shoulder height to effect laterally on the shoulder joint of the patient or above head height to effect vertically on the shoulder joint of the patient. One or more hooks 720, 715 may be used as the case may be for releasable engagement to the device 100.

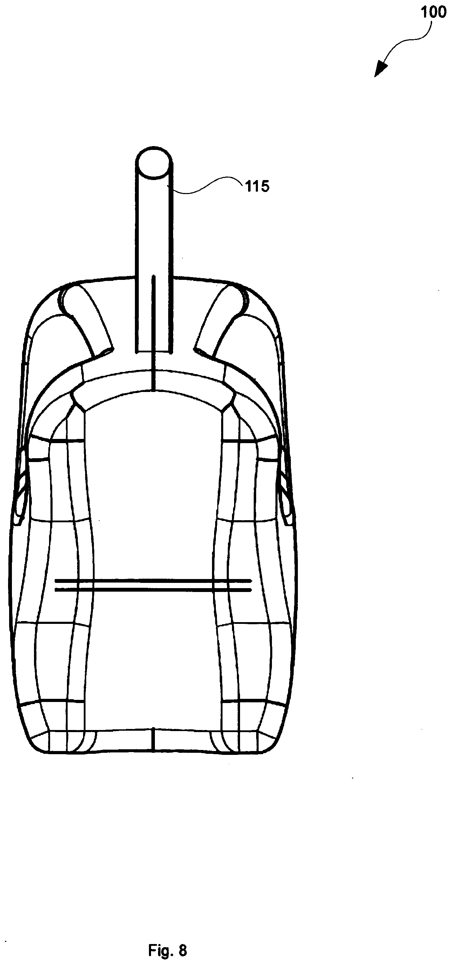

Fig. 8 shows a bottom view of the device 100 having a substantially planar bottom surface, being suitable for resting on flat surfaces.

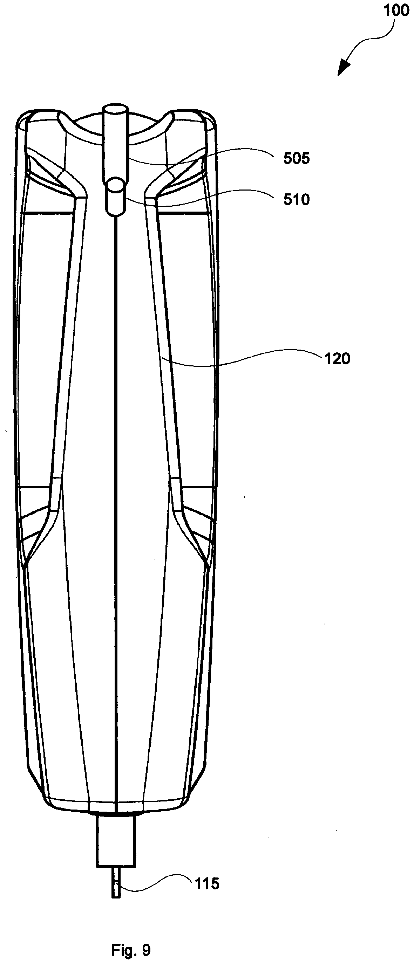

Fig. 9 shows a top view of the device 100 in this embodiment.

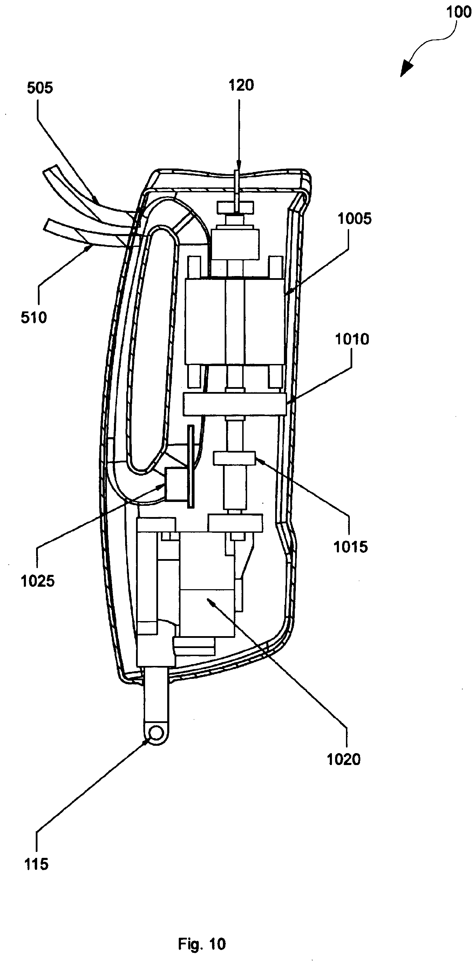

Turning now, to Fig. 10, there is shown an internal view of the device 100. The device 100 comprises a control PCB 1025. The control PCB 1025 may comprise relay switching for being suitable for 100-240v AC. The control PCB 1025 is wired to the controller (described below) or may be wireless coupled to the controller where the controller is a wireless controller.

The device 100 further comprises an electric motor 1005 coupled to a cooling fan 1010 and rotary drive coupling 1015.

The device 100 further comprises geared transmission 1020 coupled between the rotary drive coupling 1015 and the manipulation effector 115 employing a piston and shaft drive so as to impart rotary motion from the rotary drive coupling 1015 into reciprocating point-to-point motion.

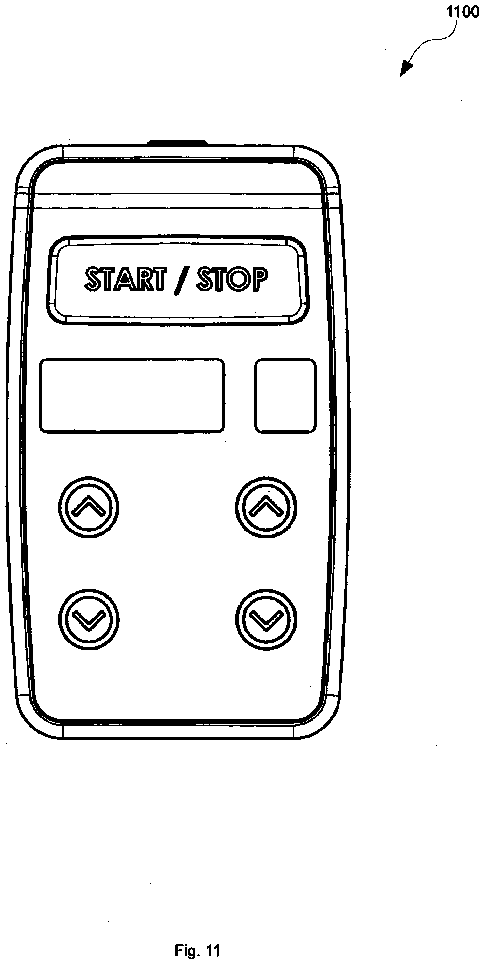

Turning now to Fig. 11, there is shown a controller 1100 for remote control of the device 100. The controller 1100 may be wired to the device using control cord 510 or may be wirelessly coupled to the device 100, by radio wave, infrared and the like.

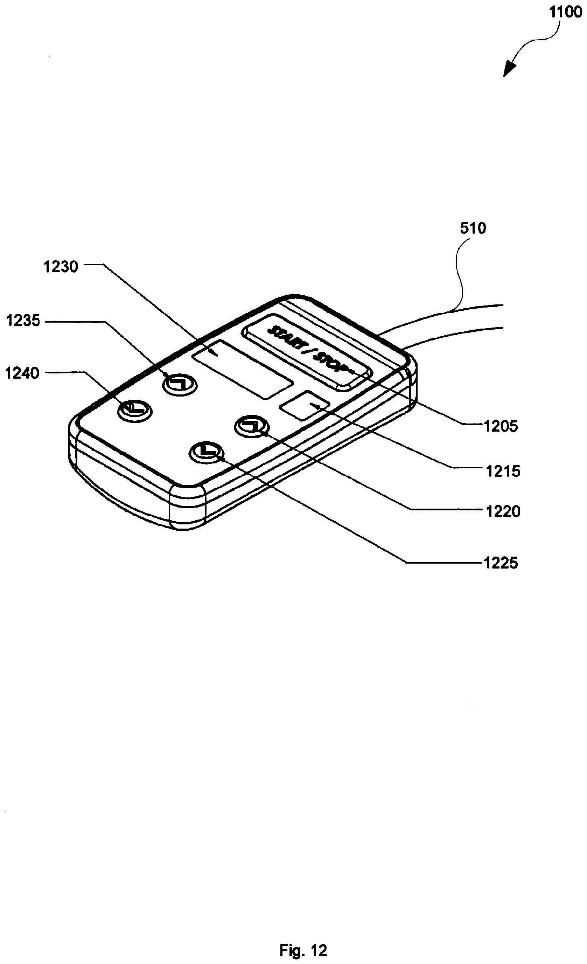

Fig. 12 shows a perspective view of the controller 1100 wherein the controller 1100 comprises a display device 1230 indicating a cycle operating time, usually in the form of a numerical countdown. The controller 1100 further comprises time control buttons having a time control button 1235 for increasing the cycle time and a time control button 1240 for decreasing the cycle time.

Similarly, the controller 1100 further comprises frequency control buttons having a frequency control button 1220 for increasing the stroke speed of the manipulation effector and a frequency control button 1240 for decreasing the stroke speed. The controller 1100 further comprises a display device 1215 for displaying the current stroke speed.

Note that in certain embodiments the controller 1100 may comprise control buttons for controlling the stroke distance of the manipulation effector.

The controller 1100 further comprises a start/stop button 1205 for activating and deactivating the device 100.

By default, the controller 1100 is adapted to use the last setting upon powerup.

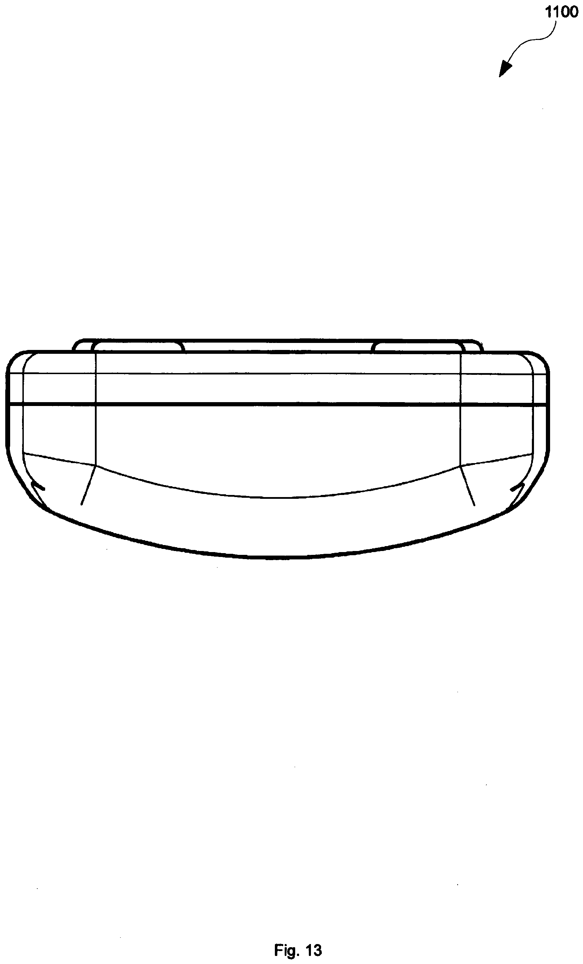

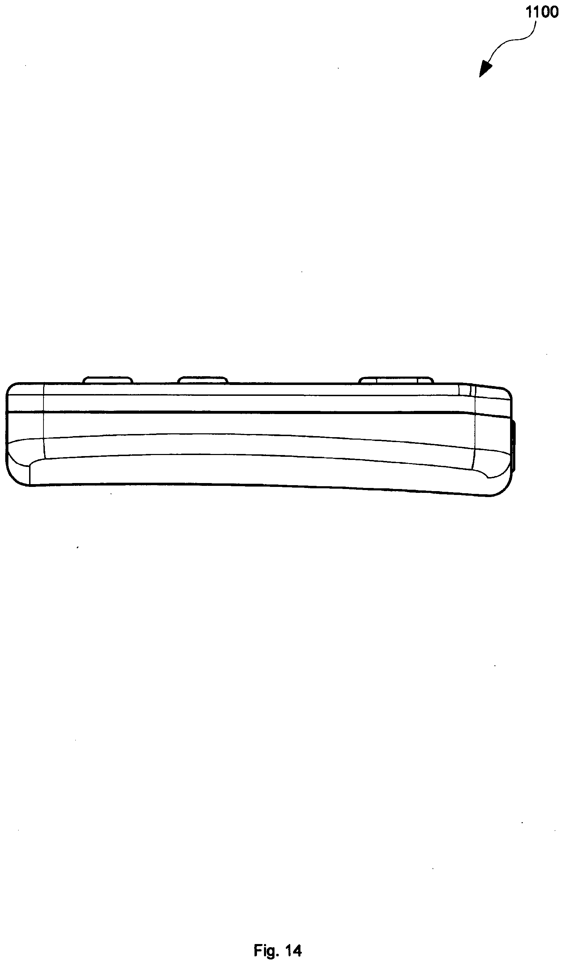

Fig. 13 shows a top view of the controller 1100 and Fig. 14 shows a bottom view of the controller 1100.

### Interpretation

### Adhesive capsulitis

Reference throughout this specification to "adhesive capsulitis" includes other disorders, such as disorders similar to adhesive capsulitis, including calcific tendinitis and the like.

### Embodiments:

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the above description of example embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the Detailed Description of Specific Embodiments are hereby expressly incorporated into this Detailed Description of Specific Embodiments, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

### Specific Details

In the description provided herein, numerous specific details are set forth. However, it is understood that embodiments of the invention may be practiced without these specific details. In other instances, well-known methods, structures and techniques have not been shown in detail in order not to obscure an understanding of this description.

### Terminology

In describing the preferred embodiment of the invention illustrated in the drawings, specific terminology will be resorted to for the sake of clarity. However, the invention is not intended to be limited to the specific terms so selected, and it is to be understood that each specific term includes all technical equivalents which operate in a similar manner to accomplish a similar technical purpose. Terms such as "forward", "rearward", "radially", "peripherally", "upwardly", "downwardly", and the like are used as words of convenience to provide reference points and are not to be construed as limiting terms.

### Different Instances of Objects

As used herein, unless otherwise specified the use of the ordinal adjectives "first", "second", "third", etc., to describe a common object, merely indicate that different instances of like objects are being referred to, and are not intended to imply that the objects so described must be in a given sequence, either temporally, spatially, in ranking, or in any other manner.

### Comprising and Including

In the claims which follow and in the preceding description of the invention, except where the context requires otherwise due to express language or necessary implication, the word "comprise" or variations such as "comprises" or "comprising" are used in an inclusive sense, i.e. to specify the presence of the stated features but not to preclude the presence or addition of further features in various embodiments of the invention.

Any one of the terms: including or which includes or that includes as used herein is also an open term that also means including at least the elements/features that follow the term, but not excluding others. Thus, including is synonymous with and means comprising.

### Scope of Invention

Although the invention has been described with reference to specific examples, it will be appreciated by those skilled in the art that the invention may be embodied in many other forms. The scope of the invention is defined by the appended claims.

### Industrial Applicability

It is apparent from the above, that the arrangements described are applicable to the theraputic and medical device industries.

## Claims

1. A device (100) for the treatment of adhesive capsulitis in a joint of a patient, the device (100) being a portable, hand-held appliance and comprising:
a) a driven manipulation effector (115) adapted to engage an arm of the patient in order to manipulate the patient's joint, wherein the device (100) is adapted such that the manipulation effector (115) moves in accordance with a point-to-point motion profile (200);
b) a driven oscillator (105), wherein the driven manipulation effector (115) is coupled to the driven oscillator (105) and adapted to manipulate the joint, and wherein the driven oscillator (105) is adapted to displace the driven manipulation effector (115) in accordance with the point-to-point motion profile (200);
c) a motor (140) coupled to a power supply (135) and adapted to drive the driven oscillator (105), and a drive shaft (120) coupling the motor (140) and the driven oscillator (105);
d) a body (110) coupling the driven oscillator (105) to the driven manipulation effector (115); and
e) an anchor (120) adapted for securement of the body to a secure object to enable the driven manipulation effector (115) to manipulate the patient's joint;
the device (100) being **characterised in that**:
the driven oscillator (105) is mechanically coupled to the drive shaft (120) at an attachment point offset from the axis of the drive shaft (120) and adapted to impart a rotational motion upon the driven oscillator (105) when the motor (140) is in use, and **in that** the manipulation effector (115) is adapted to move in a point-to-point motion profile (200) including:
i) a first phase of motion under constant acceleration;
ii) a second phase of motion under zero acceleration; and
iii) a third phase of motion under constant deceleration.

2. A device (100) as claimed in claim 1, wherein the manipulation effector (115) is mechanically coupled to the driven oscillator (105).

3. A device (100) as claimed claim 1 or 2, wherein the manipulation effector (115) is adapted to move in a point-to-point motion profile (200) comprising a displacement of between 3mm and 12mm.

4. A device (100) as claimed in any one of claims 1 to 3, wherein the manipulation effector (115) is adapted to move in a point-to-point motion profile (200) comprising a period of between 0.015s and 0.3s.

5. A device (100) as claimed in any one of claims 1 to 4, wherein the manipulation effector (115) is adapted to move in a point-to-point motion profile (200) with at least one phase of motion comprising a jerk motion.

6. A device (100) as claimed in any one of claims 1 to 5, wherein the manipulation effector (115) is adapted to move in a point-to-point motion profile (200) comprising:
i) a first phase of motion under increasing acceleration;
ii) a second phase of motion under constant acceleration;
iii) a third phase of motion under decreasing acceleration; and
iv) a fourth phase of motion under zero acceleration.

7. A device (100) as claimed in claim 6, wherein the manipulation effector (115) is adapted to move in a point-to-point motion profile (200) further comprising:
i) a fifth phase of motion under increasing deceleration;
ii) a sixth phase of motion under constant deceleration; and
iii) a seventh phase of motion under decreasing deceleration.

8. A device (100) as claimed in claim 1, wherein a drive shaft (120) comprises a bearing surface (310) having an axis offset from the axis of the drive shaft (120) and wherein the crank (305) bears against the bearing surface (310).

## Patentansprüche

1. Vorrichtung (100) zum Behandeln von adhäsiver Kapsulitis in einem Gelenk eines Patienten, wobei die Vorrichtung (100) ein tragbarer, handgehaltener Apparat ist und Folgendes umfasst:
a) einen angetriebenen Manipulationseffektor (115), der angepasst ist, einen Arm des Patienten in Eingriff zu nehmen, um das Gelenk des Patienten zu manipulieren, wobei die Vorrichtung (100) derartig angepasst ist, dass der Manipulationseffektor (115) sich in Übereinstimmung mit einem Punkt-zu-Punkt-Bewegungsprofil (200) bewegt;
b) einen angetriebenen Oszillator (105), wobei der angetriebene Manipulationseffektor (115) mit dem angetriebenen Oszillator (105) verbunden ist und angepasst ist, das Gelenk zu manipulieren, und wobei der angetriebene Oszillator (105) angepasst ist, den angetriebenen Manipulationseffektor (115) in Übereinstimmung mit dem Punkt-zu-Punkt-Bewegungsprofil (200) zu versetzen;
c) einen Motor (140), verbunden mit einer Stromversorgung (135) und angepasst den angetriebenen Oszillator (105) anzutreiben, und eine Antriebswelle (120), die den Motor (140) und den angetriebenen Oszillator (105) verbindet;
d) einen Körper (110), welcher den angetriebenen Oszillator (105) mit dem angetriebenen Manipulationseffektor (115) verbindet; und
e) eine Verankerung (120), angepasst zum Sichern des Körpers an einem sicheren Gegenstand, um es dem angetriebenen Manipulationseffektor (115) zu ermöglichen, das Gelenk des Patienten zu manipulieren;
wobei die Vorrichtung (100) **dadurch gekennzeichnet ist, dass**
der angetriebene Oszillator (105) mechanisch mit der Antriebswelle (120) an einem Befestigungspunkt von der Achse der Antriebswelle (120) versetzt verbunden ist und angepasst ist, dem angetriebenen Oszillator (105) eine Rotationsbewegung zu erteilen, wenn der Motor (140) in Betrieb ist, und dadurch, dass der Manipulationseffektor (115) angepasst ist, sich in einem Punkt-zu-Punkt-Bewegungsprofil (200) zu bewegen, welches Folgendes beinhaltet:
i) eine erste Bewegungsphase mit konstantem Beschleunigen;
ii) eine zweite Bewegungsphase ohne Beschleunigen; und
iii) eine dritte Bewegungsphase mit konstantem Verlangsamen.

2. Vorrichtung (100) nach Anspruch 1, wobei der Manipulationseffektor (115) mechanisch mit dem angetriebenen Oszillator (105) verbunden ist.

3. Vorrichtung (100) nach Anspruch 1 oder 2, wobei der Manipulationseffektor (115) angepasst ist, sich in einem Punkt-zu-Punkt-Bewegungsprofil (200), eine Versetzung zwischen 3 mm und 12 mm umfassend, zu bewegen.

4. Vorrichtung (100) nach einem der Ansprüche 1 bis 3, wobei der Manipulationseffektor (115) angepasst ist, sich in einem Punkt-zu-Punkt-Bewegungsprofil (200), eine Zeitdauer zwischen 0,015 s und 0,3 s umfassend, zu bewegen.

5. Vorrichtung (100) nach einem der Ansprüche 1 bis 4, wobei der Manipulationseffektor (115) angepasst ist, sich in einem Punkt-zu-Punkt-Bewegungsprofil (200) zu bewegen, wobei wenigstens eine Bewegungsphase eine Ruckbewegung umfasst.

6. Vorrichtung (100) nach einem der Ansprüche 1 bis 5, wobei der Manipulationseffektor (115) angepasst ist, sich in einem Punkt-zu-Punkt-Bewegungsprofil (200) zu bewegen, Folgendes umfassend:
i) eine erste Bewegungsphase mit ansteigendem Beschleunigen;
ii) eine zweite Bewegungsphase mit konstantem Beschleunigen;
iii) eine dritte Bewegungsphase mit abnehmendem Beschleunigen; und
iv) eine vierte Bewegungsphase ohne Beschleunigen.

7. Vorrichtung (100) nach Anspruch 6, wobei der Manipulationseffektor (115) angepasst ist, sich in einem Punkt-zu-Punkt-Bewegungsprofil (200) zu bewegen, ferner Folgendes umfassend:
i) eine fünfte Bewegungsphase mit zunehmendem Verlangsamen;
ii) eine sechste Bewegungsphase mit konstantem Verlangsamen; und
iii) eine siebte Bewegungsphase mit abnehmendem Verlangsamen.

8. Vorrichtung (100) nach Anspruch 1, wobei eine Antriebswelle (120) eine Auflageoberfläche (310), die eine Achse versetzt von der Achse der Antriebswelle (120) hat, umfasst und wobei die Kurbel (305) gegen die Auflageoberfläche (310) drückt.

## Revendications

1. Dispositif (100) pour le traitement de la capsulite rétractile dans une articulation d'un patient, le dispositif (100) étant un appareil portatif et comprenant :
a) un effecteur de manipulation entraîné (115) adapté pour engager un bras du patient afin de manipuler l'articulation du patient, dans lequel le dispositif (100) est adapté de sorte que l'effecteur de manipulation (115) se meut selon un profil de mouvement point à point (200) ;
b) un oscillateur entraîné (105), dans lequel l'effecteur de manipulation entraîné (115) est couplé à l'oscillateur entraîné (105) et adapté pour manipuler l'articulation, et dans lequel l'oscillateur entraîné (105) est adapté pour déplacer l'effecteur de manipulation entraîné (115) selon le profil de mouvement point à point (200) ;
c) un moteur (140) couplé à une alimentation électrique (135) et adapté pour entraîner l'oscillateur entraîné (105), et un arbre d'entraînement (120) couplant le moteur (140) et l'oscillateur entraîné (105) ;
d) un corps (110) couplant l'oscillateur entraîné (105) à l'effecteur de manipulation entraîné (115) ; et
e) un ancrage (120) adapté pour fixer le corps à un objet bien fixé afin de permettre à l'effecteur de manipulation entraîné (115) de manipuler l'articulation du patient ;
le dispositif (100) étant **caractérisé en ce que** :
l'oscillateur entraîné (105) est couplé mécaniquement à l'arbre d'entraînement (120) en un point d'attache décalé de l'axe de l'arbre d'entraînement (120) et adapté pour communiquer un mouvement de rotation à l'oscillateur entraîné (105) lorsque le moteur (140) est en utilisation, et **en ce que** l'effecteur de manipulation (115) est adapté pour se mouvoir dans un profil de mouvement point à point (200) incluant :
i) une première phase de mouvement sous accélération constante ;
ii) une deuxième phase de mouvement sous accélération nulle ; et
iii) une troisième phase de mouvement sous décélération constante.

2. Dispositif (100) selon la revendication 1, dans lequel l'effecteur de manipulation (115) est couplé mécaniquement à l'oscillateur entraîné (105).

3. Dispositif (100) selon la revendication 1 ou 2, dans lequel l'effecteur de manipulation (115) est adapté pour se mouvoir dans un profil de mouvement point à point (200) comprenant un déplacement entre 3 mm et 12 mm.

4. Dispositif (100) selon l'une quelconque des revendications 1 à 3, dans lequel l'effecteur de manipulation (115) est adapté pour se mouvoir dans un profil de mouvement point à point (200) comprenant une période entre 0,015 s et 0,3 s.

5. Dispositif (100) selon l'une quelconque des revendications 1 à 4, dans lequel l'effecteur de manipulation (115) est adapté pour se mouvoir dans un profil de mouvement point à point (200) avec au moins une phase de mouvement comprenant un mouvement de secousse.

6. Dispositif (100) selon l'une quelconque des revendications 1 à 5, dans lequel l'effecteur de manipulation (115) est adapté pour se mouvoir dans un profil de mouvement point à point (200) comprenant :
i) une première phase de mouvement sous accélération croissante ;
ii) une deuxième phase de mouvement sous accélération constante ;
iii) une troisième phase de mouvement sous accélération décroissante ; et
iv) une quatrième phase de mouvement sous accélération nulle.

7. Dispositif (100) selon la revendication 6, dans lequel l'effecteur de manipulation (115) est adapté pour se mouvoir dans un profil de mouvement point à point (200) comprenant en outre :
i) une cinquième phase de mouvement sous décélération croissante ;
ii) une sixième phase de mouvement sous décélération constante ; et
iii) une septième phase de mouvement sous décélération décroissante.

8. Dispositif (100) selon la revendication 1, dans lequel un arbre d'entraînement (120) comprend une surface d'appui (310) ayant un axe décalé de l'axe de l'arbre d'entraînement (120) et dans lequel la manivelle (305) appuie contre la surface d'appui (310).
